# EUROPEAN PATENT SPECIFICATION

(11) **EP 3 003 431 B1**
(45) Date of publication and mention of the grant of the patent: **02.03.2022**
(21) Application number: 14736969.8
(22) Date of filing: 26.05.2014
(51) Int. Cl.: A61M 5/14, A61M 5/168, A61M 5/142, A61M 5/178, G21F 5/018, A61M 5/00

(54) **CARTRIDGE FOR A RADIOPHARMACEUTICAL, SHIELDED CONTAINER FOR SAID CARTRIDGE AND CORRESPONDING APPARATUS FOR INFUSION OF A RADIOPHARMACEUTICAL DOSE TO A PATIENT**
KARTUSCHE FÜR EIN RADIOPHARMAZEUTIKUM, ABGESCHIRMTER BEHÄLTER FÜR DIE KARTUSCHE UND ENTSPRECHENDE VORRICHTUNG ZUR INFUSION EINER RADIOPHARMAZEUTISCHEN DOSIS BEI EINEM PATIENTEN
CARTOUCHE POUR UN PRODUIT RADIOPHARMACEUTIQUE, CONTENANT PROTÉGÉ POUR LADITE CARTOUCHE ET APPAREIL CORRESPONDANT POUR LA PERFUSION D'UNE DOSE DE PRODUIT RADIOPHARMACEUTIQUE DANS UN PATIENT

(30) Priority: 24.05.2013 IT BO20130256
(43) Date of publication of application: 13.04.2016
(73) Proprietor: Comecer S.p.A., Castel Bolognese (RA) (IT)
(72) Inventor: GUIDI, Giacomo, 47020 Longiano (IT); ZANELLI, Alessia, 48018 Faenza (IT)
(74) Representative: Boggio, Luigi
(86) International application number: PCT/IB2014/061729
(87) International publication number: WO 2014/188401

(56) References cited:
- EP-A1- 0 102 121
- WO-A1-2009/106977
- WO-A2-02/073628
- US-A- 4 188 539
- US-A1- 2005 253 085
- US-A1- 2010 030 009

## Description

The present invention relates to a cartridge for a radiopharmaceutical, to a shielded container for containing said cartridge, and to a corresponding apparatus for administration by infusion of a radiopharmaceutical dose to a patient.

The preparation of doses of radiopharmaceuticals and their administration by infusion to patients call for adequate precautions to reduce as far as possible the risk of radiation emitted by the radiopharmaceuticals being absorbed by the body of the person responsible for the preparation and administration of the pharmaceuticals.

The preparation of a radiopharmaceutical dose basically consists in filling a purposely designed container, for example a flask or a syringe, with a pre-set amount of radiopharmaceutical and is carried out in an environment shielded from radiation, and the staff is equipped with a purposely designed protective clothing. The container with the radiopharmaceutical dose is then inserted and closed in a shielded container and taken to the infusion room.

Normally, preparation and transport are the least critical steps from the standpoint of dispersion of radiation from the flask or syringe, unlike the administration step, where the shielded container is to be opened to connect the container containing the radiopharmaceutical dose to the infusion tubing. Moreover, frequently the radiopharmaceutical dose is infused by means of a manually operated syringe, with consequent continuous exposure of the staff to radiation.

The United States patent application No. US 2005/0253085 A1 describes a device for producing a fluid containing a radioactive constituent, comprising a shielded chamber with an opening for receiving an isotope container housing a radioactive isotope; a chamber closure adapted for cooperating with and closing the chamber opening; a first fluid port comprising a first hollow needle projecting into the shielded chamber from the chamber closure for fluid communication with the isotope container; a second fluid port comprising a second hollow needle projecting into the shielded chamber from the closed end of the chamber opposite the chamber closure for fluid communication with the isotope container; first and second compressible buffers mounted so as to surround at least partially the respective first and second hollow needles, each buffer providing an outer surface for contact with opposed ends of the isotope container; and a spacer of a predetermined thickness associated with one or each of the first and second compressible buffers for determining the positioning of the isotope container within the shielded chamber.

The United States patent application No. US 2010/0030009 A1 describes a medical unit, which includes a shielded enclosure in which are accommodated: elements for supporting a container including a source or a generator of injectable radioactive product, elements for supporting a syringe, a device of the activimeter type, and a system of pipes associated with at least one valve. The syringe support, the valve and the radioactive source support are arranged vertically relative to one another, respectively from top to bottom, the syringe support being arranged to support the syringe with the plunger thereof oriented upward. The valve and the syringe plunger can be operated so as to ensure the withdrawal, dilution and injection operations.

The European patent application No. EP 2179758 A2 describes an apparatus for infusion of radiopharmaceuticals that attempts to overcome the drawbacks listed previously. Said apparatus is provided with a portable automatic infusor device that can be connected to the infusion tubing. The automatic infusor device comprises a purposely manufactured syringe inserted in a tailor-made shielded container, from which the capillary tube provided with connection plug or valve and the piston of the syringe exit. The syringe is coupled to an electronically controlled linear actuator, which is able to move, in opposite directions, the piston of the syringe so as to draw the radiopharmaceutical into the syringe, during preparation of the dose, and expel the radiopharmaceutical from the syringe, during administration of the dose. During administration of the radiopharmaceutical, the automatic infusor device is to be inserted in a shielded pocket mounted on the support of the tubing of the infusion apparatus and there is no need for staff to remain close to the automatic infusor device that contains the radiopharmaceutical. Hence, the infusion apparatus according to the patent application No. EP 2179758 A2 reduces the risk of absorption of radiation by staff responsible for carrying out administration of radiopharmaceuticals.

However, the apparatus described above still presents multiple disadvantages. First of all, at the end of administration, there always remain traces of radiopharmaceutical in the syringe. Hence, before being thrown away, the syringe must be washed thoroughly. This is one of the reasons why the syringe of the infusor device described above cannot be conveniently used as disposable device. The other reason is that said syringe is not of a standard type and is hence per se costly. Other disadvantages derive from the constructional details of the automatic infusor device, which is per se costly and could introduce some elements of unreliability.

The aim of the present invention to provide an apparatus for infusion of a radiopharmaceutical dose to a patient that will be free from the drawbacks described above and, at the same time, will be easy and economically advantageous to produce.

In accordance with the present invention, a cartridge for containing and for administration by infusion to a patient of a fluid radiopharmaceutical, a shielded container configured to contain a cartridge, and an apparatus for administration by infusion of a radiopharmaceutical dose to a patient are provided according to what is defined in the annexed claims.

For a better understanding of the present invention, a preferred embodiment is now described, purely by way of nonlimiting example and with reference to the attached drawings, wherein:
- Figure 1 illustrates, according to a longitudinal sectional view, the cartridge for containing a radiopharmaceutical provided according to the present invention;
- Figure 2 illustrates, according to a longitudinal sectional view, the shielded container of the present invention, open and without the cartridge;
- Figures 3 and 4 illustrate, according to the same view of Figure 2, the shielded container with the cartridge of Figure 1 inside, in two different configurations of use;
- Figures 5 and 6 illustrate the shielded container in the two configurations of use of Figures 3 and 4, but according to a perspective view from outside; and
- Figure 7 illustrates, in front view, the apparatus for infusion of a radiopharmaceutical dose provided according to the teachings of the present invention.

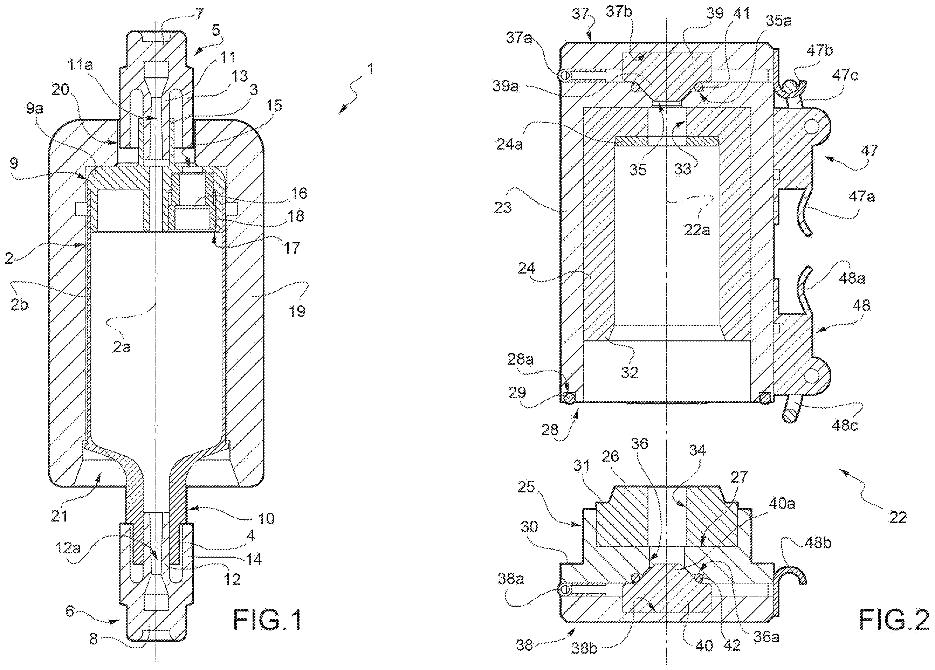

In Figure 1, designated as a whole by 1 is the cartridge for containing a liquid radiopharmaceutical, made according to the teachings of the present invention. The cartridge 1 comprises a container 2 for the radiopharmaceutical, which is made of a plastic material suitable for containing injectable pharmaceuticals and has, at its two longitudinal ends, two respective mouths 3 and 4. The container 2 comprises two plugs 5 and 6, each of which hermetically seals a respective mouth 3, 4 and comprises in a central position a respective diaphragm 7, 8, which can be perforated by a needle to enable the latter to penetrate into the container 2 in order to fill or empty the cartridge 1. The container is without any plunger or pusher inside it.

The container 2 has a cylindrical shape, and in particular the shape of a flask having a bottom 9 and a neck 10. The container 2 is sized for containing up to 100 ml of liquid pharmaceutical. The mouth 3 is made in the wall 9a of the bottom 9 that is transverse to the longitudinal axis 2a of the container 2, and the mouth 4 coincides with the mouth of the neck 10. Each mouth 3, 4 has a circular shape and projects towards the outside of the container 2. The mouth 4 coincides, in effect, with the mouth of the neck 10. The mouth 3 is centred in the bottom 9. Hence, the mouths 3 and 4 share the axis 2a. The container 2 is provided in two pieces constituted by a flask body 2b without bottom, and by the bottom 9, hermetically coupled to the body of the flask 2b. The mouth 3 is to be used as inlet mouth for the liquids to be introduced into the cartridge 1, whereas the mouth 4 is to be used as outlet mouth for the liquids to be drawn out of the cartridge 1, in so far as the shape of the neck 10, when the cartridge 1 faces with the neck 10 downwards as in Figure 1, favours complete emptying of the cartridge 1. Consequently, the diaphragm 7 is the inlet diaphragm, and the diaphragm 8 is the outlet diaphragm.

Each plug 5 and 6 is made of PVC, except for the respective diaphragm 7, 8, which is made of rubber. Each diaphragm 7, 8 is set at the top end of the plug 5, 6 and is transverse to the longitudinal axis of the respective plug 5, 6, which coincides with the axis 2a. Each plug 5, 6 comprises a cylindrical stem 11, 12, which hermetically engages the corresponding mouth 3, 4 and has an internal longitudinal hole 11a, 12a, which terminates against the corresponding diaphragm 7, 8, and an outer cylindrical portion 13, 14, which is set around, and at a certain distance from, the respective stem 11, 12 so as to surround the mouth 3, 4 at least partially and without coming into contact therewith.

The container 2 moreover has a ventilation hole 15, which is provided in the wall 9a of the bottom 9 and is closed by a hydrophobic membrane 16 to enable passage of air during filling or emptying of the cartridge 1 through just one of the two mouths 3 and 4 and at the same prevent passage of liquids. In particular, the bottom 9 has a seat 17, which communicates with the ventilation hole 15 and hermetically houses a sleeve 18 having inside it the hydrophobic membrane 16 to prevent the liquid from coming out of the container 2. Advantageously, the hydrophobic membrane 16 has also a microfiltering function to prevent impurities from entering the container 2 with entry of air from the ventilation hole 15.

Preferably, but not necessarily, the cartridge 1 comprises a casing 19 that is made of radiation-shielding material and that adheres on the outside the container 2. The material of the casing 19 is able to absorb the radiation emitted by the radiopharmaceutical present inside the container 2. For example, the material of the casing 19 is tungsten. The casing 19 has a cylindrical shape and has, at its two longitudinal ends, two respective openings 20 and 21 made in positions corresponding to the mouths 3 and 4. The plug 5 engages the opening 20 with play, projecting at least in part therefrom, whereas the neck 10 of the container 2 and the plug 6 project from the opening 21. The opening 21 has a diameter such as to enable insertion of the container 2 in the casing 19 during assemblage of the cartridge 1. The opening 20 is sized for leaving at least part of the ventilation hole 15 exposed. The casing 19 is sized so as to adhere on the outside at least to the side wall of the container 2.

In Figure 2, designated as a whole by 22 is the shielded container for the cartridge 1, provided according to the teachings of the present invention. The shielded container 22 comprises a cup-shaped supporting body 23, a cup-shaped inner body 24, which is made of radiation-shielding material and adheres completely, i.e., with its own bottom and its own side wall, to the inside of the cup-shaped supporting body 23 and is designed to house the cartridge 1, a main lid 25 for closing the cup-shaped supporting body 23, and an inner lid 26, which is made of radiation-shielding material and is fixed with respect to the main lid 25 for closing the cup-shaped inner body 24 when the main lid 25 closes the cup-shaped supporting body 23. In particular, the main lid 25 comprises an internal seat 27 engaged with interference by the inner lid 26. In Figure 2, the shielded container 22 is illustrated open, i.e., with the main lid 25 moved away from the cup-shaped supporting body 23 to enable insertion of the cartridge 1 (not illustrated).

Preferably, the cup-shaped supporting body 23 and the main lid 25 are made of aluminium. The cup-shaped inner body 24 and the inner lid 26 are made, for example, of tungsten.

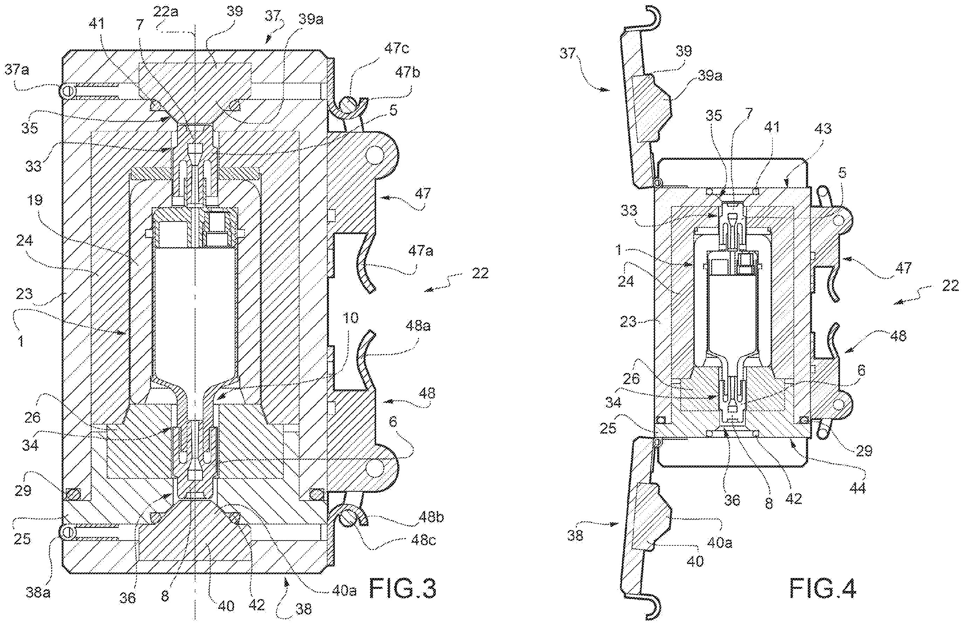

With reference to Figures 2 and 3, the rim 28 of the mouth of the cup-shaped supporting body 23 has a groove 28a in which a hermetic sealing element (O-ring) 29 is housed. The main lid 25 has a shoulder 30 that bears upon the rim 28, when the lid 25 closes the cup-shaped body 23, compressing the sealing element 29 and thus guaranteeing hermetic seal between the cup-shaped body 23 and the lid 25. The shoulder 30 has a right-angle profile. The inner lid 26 comprises a shoulder 31 having an obtuse-angle profile that bears upon the rim 32 of the mouth of the cup-shaped inner body 24, the rim 32 having a profile complementary to that of the shoulder 31. The two cup-shaped bodies 23 and 24 have a cylindrical shape, and the two lids 25 and 26 have a circular shape. Consequently, the rims 28 and 32, the groove 28a, and the hermetic sealing element 29 have annular shapes, and the internal seat 27 has a cylindrical shape. The views of Figures 2 to 4 are sections along the longitudinal axis of symmetry 22a of the shielded container 22, which coincide with the longitudinal axes of the bodies 23 and 24.

The bottom of the cup-shaped inner body 24 and the inner lid 26 have respective housing openings 33 and 34, each of which can be completely engaged, in use, by a respective plug 5, 6. The housing openings 33 and 34 are constituted by respective through holes. The bottom of the cup-shaped supporting body 23 and the main lid 25 have respective access openings 35 and 36, each of which is substantially aligned to a respective housing opening 33, 34 to enable, in use, passage of a corresponding needle (not illustrated) for insertion of the needle itself into the diaphragm 7, 8 of the plug 5, 6 positioned in the housing opening 33, 34. In other words, each access opening 35, 36 enables passage of the needle that is to perforate the diaphragm 7, 8 of the plug 5, 6 positioned in the housing opening 33, 34 that is aligned to the access opening 35, 36 itself. The access openings 35 and 36 are constituted by respective through holes flared outwards.

The cup-shaped inner body 24 is sized so as to house, when it is closed with the inner lid 26, the cartridge 1 including the casing 19, possibly without any transverse play. The thickness of the inner lid 26 is sized so that the length of its own housing opening 34 will be sufficient to contain the plug 6, i.e., the one that closes the neck 10 of the cartridge 1 and that projects further from the casing 19 of the cartridge 1. A spacer ring 24a set on the bottom of the cup-shaped inner body 24 enables reduction of the axial play of the cartridge 1. Hence, the cartridge 1 must be inserted in the shielded container 22 with the neck 10 on the side of the main lid 25.

The shielded container 22 comprises two auxiliary lids 37 and 38, a first one of which is hinged on the outside of the bottom of the cup-shaped supporting body 23 by means of a hinge 37a and the second of which is hinged on the outside of the main lid 25 by means of a hinge 38a. Fixed on the inner face of each auxiliary lid 37, 38 is a respective plug 39 and 40 made of radiation-shielding material, for example tungsten, designed to close a respective access opening 35 and 36. The bodies of the auxiliary lids 37 and 38 are made of the same material as that of the cup-shaped supporting body 23 and of the main lid 25, respectively, namely, aluminium. Each auxiliary lid 37, 38 comprises a respective internal seat 37b, 38b engaged with interference by the head of the respective plug 39, 40. Each plug 39, 40 has a closing portion 39a, 40a having a frustoconical shape designed to couple with the flared shape of the respective access opening 35, 36. Each access opening 35, 36 has a respective external groove 35a, 36b, housed in which is a respective hermetic annular sealing element (O-ring) 41, 42, which is compressed by the closing portion 39a, 40a of the corresponding plug 39, 40 when the corresponding auxiliary lid 37, 38 is closed in order to guarantee hermetic closing of the access opening 35, 36.

Figure 3 illustrates the shielded container 22 completely closed, i.e., with the main lid 25 and the auxiliary lids 37 and 38 closed. The configuration illustrated in Figure 3 is the one to be used for transport of the cartridge 1 from the place where it is prepared to a place where infusion is carried out. Figure 4 illustrates, instead, the shielded container 22 in the configuration to be used in the place where infusion is carried out, in which the two auxiliary lids 37 and 38 are open to enable two needles connected to an infusion tubing to be inserted, passing through the two access openings 35 and 36, into the diaphragms 7 and 8 of the cartridge 1, as will be explained in greater detail in the sequel.

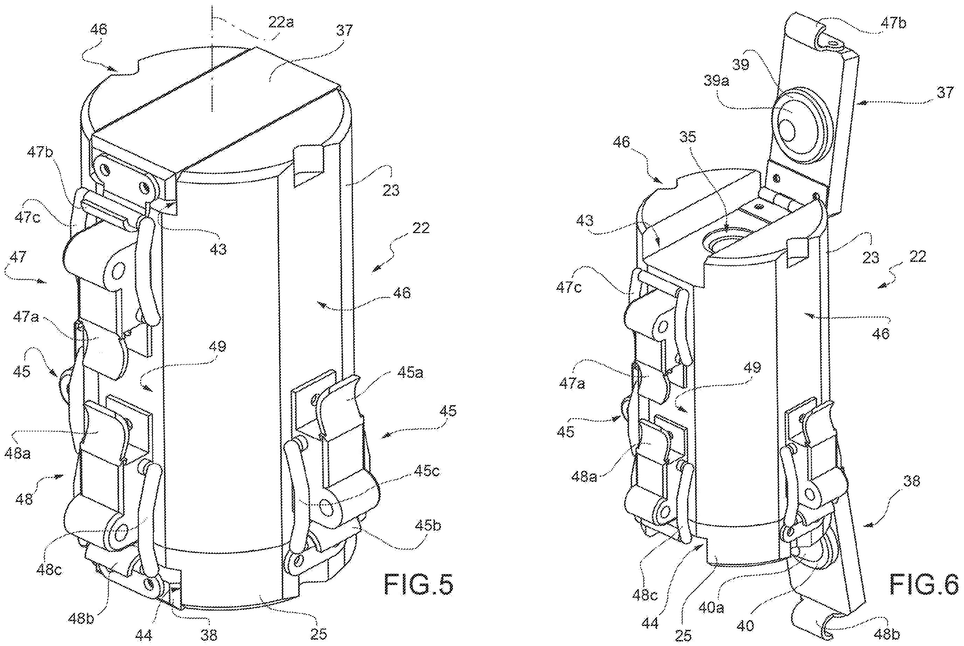

With reference to Figures 5 and 6, which illustrate the shielded container 22 in the two configurations of use of Figures 3 and 4, but according to a perspective view from outside, both of the auxiliary lids 37 and 38 have substantially rectangular shapes and are set in respective seats 43 and 44 having a complementary shape made in the bottom of the cup-shaped supporting body 23 and in the main lid 25, respectively.

The shielded container 22 comprises a pair of lever closing mechanisms, both of which are designated by 45 and just one of which is clearly visible in Figures 5 and 6, which have the respective levers 45a hinged on two respective outer longitudinal surface portions 46 of the cup-shaped supporting body 23 that are parallel to the axis 22a, and diametrally opposite with respect thereto, and the respective hooks 45b fixed on the outside of the main lid 25, in diametrally opposite positions thereof, to secure the main lid 25 against the cup-shaped supporting body 23. The shielded container 22 further comprises an additional pair of lever closing mechanisms 47 and 48, visible also in Figures 2 to 4, which have the respective levers 47a and 48a hinged on, and aligned along, an outer longitudinal surface portion 49 of the cup-shaped supporting body 23 that lies in a plane substantially perpendicular to the planes of the surface portions 46, and the respective hooks 47b and 48b each fixed on a respective auxiliary lid 37, 38, in a position opposite to the corresponding hinge 37a, 38a (Figures 2 and 3) of the lid 37, 38 to ensure closing of the auxiliary lids 37 and 38.

The lever closing mechanisms 45, 47 and 48 are of the same known type, i.e., of the type having a respective engagement eyelet 45c, 47c, 48c designed to couple with the corresponding hook 45b, 47b, 48b to pull it towards itself following upon a movement of the lever 45a, 47a, 48a.

When the main lid 25 and the auxiliary lids 37 and 38 are closed (Figures 3 and 5), the shielded container 22 is hermetically sealed, thanks to the hermetic sealing element 29, 41, 42, and is such as to shield the radiation emitted by the radiopharmaceutical, thanks to the double shielding casing having a considerable thickness, which is constituted, inside, by the casing 19 of the cartridge 1 and, outside, by the cup-shaped inner body 26 closed by the inner lid 24 and by the plugs 39 and 40 of the container 2. The casing 19 has a thickness comprised between 3 and 10 mm. The cup-shaped inner body 24 has a thickness comprised between 8 and 15 mm. The inner lid 26 has an axial thickness, measured along the axis 22a, comprised between 3 and 20 mm. The plugs 39 and 40 have an axial thickness, once again measured along the axis 22a, comprised between 3 and 20 mm.

According to a variant (not illustrated) of the present invention, the cartridge is without the casing 19, and the cup-shaped inner body 24 has a thickness that is preferably greater so as to house, when it is closed with the inner lid 26, the container 2 of the cartridge 1.

The shielded container 22 is hence a portable shielded container suitable for transport of the cartridge 1 in safety conditions from a place where it is prepared to a place where infusion is carried out and, at the same time, for direct use during infusion of the radiopharmaceutical, thanks to the auxiliary lids 37 and 38 that can be easily opened, which enable access to the perforable diaphragms 7 and 8 of the cartridge 1 with the minimum risk of escape of radiation from the cartridge 1.

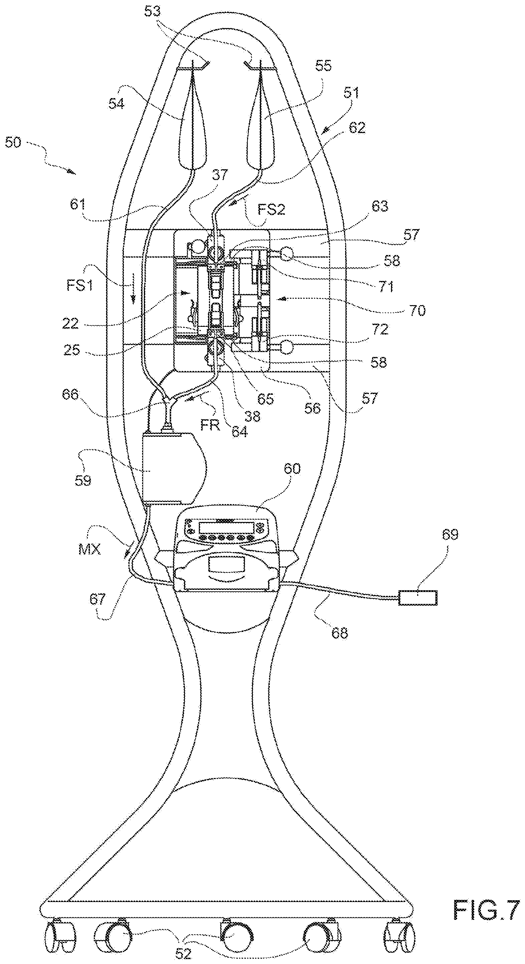

In Figure 7, designated by 50 is the apparatus for infusion of a radiopharmaceutical dose according to the present invention. The apparatus 50 comprises: the shielded container 22 with the cartridge 1 inside; a supporting frame 51, which is constituted by a tubular element curved according to a substantially vertical oval shape and is provided, at the base, with wheels 52 and, at a top end, with hooks 53 for hanging two bags 54 and 55, both containing a saline solution; a secondary frame 56, which is fixed on two horizontal cross members 57 of the frame 51 and comprises a double fork 58 for withholding, in a releasable way, the shielded container 22 in a vertical position, i.e., with the axis 22a oriented vertically; a drip chamber 59, enclosed in a box, and a peristaltic pump 60, both of which are fixed to the supporting frame 51; and a set of tubes that form the infusion tubing for connecting the bags 54 and 55 and the cartridge 1 (not visible in Figure 1) to the drip chamber 59 and the latter to a venous access of the patient (not illustrated), optionally using the pump 60.

The shielded container 22 is withheld by the double fork 58 with the main lid 25 downwards so that the cartridge 1 will be oriented with the neck 10 downwards. The auxiliary lids 37 and 38 are open to allow access to the perforable diaphragms 7 and 8 (which are not visible in Figure 7).

The infusion tubing of the apparatus 50 comprises: a tube 61 connected with a first end to the bag 54 for supplying a first flow of saline solution FS1; a tube 62 connected with a first end to the other bag 55 and provided, at the other end, with a needle 63 inserted, through the two openings 35 and 33 of the shielded container 22, into the diaphragm 7 of the cartridge 1 (not visible in Figure 7), i.e., the diaphragm in the top position, for supplying a second flow of saline solution FS2 to the cartridge 1; a tube 64 with a first end provided with a needle 65 inserted, through the openings 36 and 34 of the shielded container 22, into the other diaphragm 8 of the cartridge 1 (not visible in Figure 7), i.e., the diaphragm in the bottom position, so as to draw out a flow of radiopharmaceutical FR from the cartridge 1 as a result of the second flow of saline solution FS2 that enters the cartridge 1; a dual-inlet connector 66, which connects the other ends of the tube 61 and of the tube 64 to the inlet of the drip chamber 59 for supplying, to the latter, a mixture MX obtained by mixing the first flow of saline solution FS1 with the flow of radiopharmaceutical FR; a tube 67, which connects the outlet of the drip chamber 59 with the inlet of the pump 60; and a last tube 68 for connecting the outlet of the pump 60 to a terminal device 69 provided with a needle that is applied to the venous access of the patient.

The pump 60 sucks in the mixture MX from the drip chamber 59 so as to force outlet of the flow of radiopharmaceutical FR from the cartridge 1. In other words, the pump 60 enables the radiopharmaceutical to be drawn out of the cartridge 1 by forced suction. The pump 60 moreover enables precise adjustment of the infusion rate.

According to an alternative operating mode (not illustrated), the pump 60 is not used, and the tube 67 is directly connected to the terminal device 69. In this case, the pharmaceutical exits by gravity from the cartridge 1 as a result of the weight of the second flow of saline solution FS2 that enters the cartridge 1. This is made possible by the difference in level existing between the various parts of the apparatus 50. In particular, the bags 54 and 55 hang with their respective bottoms at a certain height H1 from the ground. The shielded container 22 is withheld in such a way that it is entirely below a height H2 from the ground less than the height H1. The drip chamber 59 is fixed in such a way that it is below a height H3 less than the height H2. The venous access of the patient must be located at a height from the ground less than the height H3. In the gravity operating mode, the infusion rate is adjusted by acting manually on the flow regulator with which the drip chamber is normally provided.

It should be noted that also the operating mode that uses the pump 60 benefits from the differences in level existing between the various parts of the apparatus 50.

It should moreover be noted that the flow of saline solution FS1 is the so-called "priming flow", necessary to prevent entry of air bubbles into the venous access. The other flow of saline solution FS2 is used also for flushing completely both the cartridge 1 and the tubes connected downstream from the residue of radiopharmaceutical, at the end of infusion of the radiopharmaceutical dose.

With reference once again to Figure 7, the apparatus 50 further comprises a guide device 70 mounted on the secondary frame 56 to facilitate centring and manual insertion of the needle 63 into the perforable diaphragm 7 and of the needle 65 into the perforable diaphragm 8. In particular, the guide device 70 comprises two mobile arms 71 and 72, which comprise respective gripping means, the first one for gripping the needle 63 and the second for gripping the needle 65, and which are mobile so that they can slide along a vertical axis so as to slide the needles 63 and 65 out of respective caps and insert the needles 63 and 65 in the corresponding diaphragms 7 and 8 of the cartridge 1 and so that they can turn about said vertical axis so as to centre the needles 63 and 65 in the corresponding diaphragms 7 and 8 prior to insertion. In Figure 7, the guide device 70 is illustrated in a condition of non-use in a resting position thereof.

Even though the invention described above refers in particular to a very precise example of embodiment, it is not to be deemed as being limited to said example of embodiment, falling within its scope all the variations, modifications, or simplifications that would be evident to the person skilled in the sector, such as for example the substitution of the two bags 54 and 55 with a single bag connected to the tubes 61 and 62, the shape of the container 2 of the cartridge 1, the materials and the thicknesses of the casing 19, of the cup-shaped inner body 24 and of the inner lid 26, and the type of closing mechanisms of the lids 25, 37 and 38.

## Claims

1. A cartridge for containing a fluid radiopharmaceutical and for its administration by infusion to a patient, the
cartridge (1) comprising: a container (2) for the radiopharmaceutical, which is made of plastic material and has, at its two longitudinal ends, two respective mouths (3, 4); a casing (19) for the container (2), which is made of radiation-shielding material; and two plugs (5, 6), each of which hermetically seals a respective mouth (3, 4) and comprises a respective diaphragm (7, 8) that can be perforated by a needle to enable the latter to penetrate into the container (2) in order to fill or empty the cartridge (1); the container (2) moreover having a ventilation hole (15) closed by a hydrophobic membrane (16) for the passage of air during filling or emptying of the cartridge (1); the casing (19) adhering externally to said container (2) and having, at its two longitudinal ends, respective openings (20, 21) made in positions corresponding to said two mouths (3, 4), from which said plugs (5, 6) project at least partially.

2. The cartridge according to Claim 1, wherein said ventilation hole (15) is made in a wall (9a) of said container (2) transverse to a longitudinal axis (2a) of the container (2) .

3. The cartridge according to Claim 1 or Claim 2, wherein said container (2) is shaped like a flask that has a bottom (9) and a neck (10); a first one of said mouths (3) and said ventilation hole (15) being made in said bottom (9) and the second mouth (4) coinciding with the mouth of said neck (10).

4. The cartridge according to any one of Claims 1 to 3, wherein said container (2) is without any plunger or pusher inside it.

5. A shielded container configured to contain a cartridge according to any one of Claims 1 to 4; the shielded container (22) comprising: a cup-shaped supporting body (23); a cup-shaped inner body (24), which is made of radiation-shielding material, adheres completely inside to the cup-shaped supporting body (23) and is designed to house the cartridge (1); a main lid (25) for closing the cup-shaped supporting body (23), and an inner lid (26), which is made of radiation-shielding material and is fixed with respect to the main lid (25) for closing the cup-shaped inner body (26) when the main lid (25) closes the cup-shaped supporting body (23); the bottom of the cup-shaped inner body (24) and the inner lid (26) having respective first openings (33, 34), each of which can be engaged, in use, by a respective one of said plugs (5, 6) of the cartridge (1); the bottom of the cup-shaped supporting body (23) and the main lid (25) having respective second openings (35, 36), each of which is substantially aligned to a respective first opening (33, 34) to enable, in use, the passage of a corresponding needle for insertion thereof in the perforable diaphragm (7, 8) of the plug (5, 6) that engages the first opening (33, 34).

6. The shielded container according to Claim 5, and comprising two auxiliary lids (37, 38), which are hinged, a first one (37), on the outside of said bottom of the cup-shaped supporting body (23) and, the other, on the outside of said main lid (25) and are provided, on their own inner faces, with respective portions (39, 40) made of radiation-shielding material, each of which has the purpose of closing a respective one of said second openings (35, 36).

7. The shielded container according to Claim 5 or Claim 6, and comprising a first pair of lever closing mechanisms (45), which have the respective levers (45a) hinged on respective outer surface portions (46) of said cup-shaped supporting body (23) that are diametrally opposite with respect to a longitudinal axis (22a) of the cup-shaped supporting body (23) and have the respective hooks (45b) fixed on the outside of said main lid (25) to secure the latter against the cup-shaped supporting body (23).

8. The shielded container according to any one of Claims 5 to 7, wherein the mouth of said cup-shaped supporting body (23) has first hermetic-sealing means (29) designed to be pressed by said main lid (25) when it is in the position for closing the cup-shaped supporting body (23) to guarantee hermetic sealing between the main lid (25) and the cup-shaped supporting body (23).

9. The shielded container according to any one of Claims 6 to 8, wherein each of said second openings (35, 36) is provided with respective hermetic-sealing means (41, 42) designed to be pressed by said auxiliary lids (37, 38) when they are in the position for closing the second openings (35, 36) to guarantee hermetic closing of the latter.

10. The shielded container according to any one of Claims 6 to 9, and comprising a second pair of lever closing mechanisms (47, 48), which have the respective levers (47a, 48a) aligned along, and hinged on, a further outer surface portion (49) of the cup-shaped supporting body (23) and the respective hooks (47b, 48b), each of which is fixed on a respective auxiliary lid (37, 38) to ensure closing of the auxiliary lids (37, 38).

11. An apparatus for administration by infusion of a radiopharmaceutical dose to a patient, comprising: a cartridge according to any one of Claims 1 to 4 and containing said radiopharmaceutical dose; a shielded container (22) according to any one of Claims 5 to 10 and containing said cartridge (1); supporting means (51) for supporting saline-solution containing means (54, 55) and said shielded container (22); a first tube (62) connected to the saline-solution containing means (55, 54) and provided with a first needle (63) to be inserted, through the corresponding first and second openings (33, 35) of the shielded container (22), into a first perforable diaphragm (7) of the cartridge (1) for supplying the saline solution to the cartridge (1); a second tube (64) provided with a second needle (65) to be inserted, through the other corresponding first and second openings (33, 35) of the shielded container (22), into the other perforable diaphragm (8) of the cartridge (1) for drawing out a flow of radiopharmaceutical (FR); a dual-inlet connector (66) connected to the second tube (64) and, by means of a third tube (61), to the saline-solution containing means (54, 55) for supplying a mixture (MX) of saline solution and radiopharmaceutical; and a drip chamber (59), connected between the outlet of the dual-inlet connector (66) and a venous access of the patient for infusion of the mixture (MX) into the patient.

12. The apparatus according to Claim 11, wherein said cartridge (1) is a cartridge according to Claim 4 and said supporting means (51) comprise retaining means (58) for withholding, in a releasable way, said shielded container (22), with said cartridge (1) oriented with said neck (10) facing downwards.

13. The apparatus according to Claim 11 or Claim 12, wherein said supporting means (51) support said saline-solution containing means (54, 55) at a first height (H1) from the ground and said shielded container (22) at a second height (H2) from the ground, the first height (H1) and the second height (H2) being greater than the height from the ground of said venous access and the second height (H2) being less than the first height (H1) so that said flow of radiopharmaceutical (FR) drops out of the cartridge (1) as a result of the weight of the saline solution (FS2) that enters the cartridge (1).

14. The apparatus according to any one of Claims 11 to 13 and comprising a peristaltic pump (60) connected between the outlet of said drip chamber (59) and said venous access for sucking in said mixture (MX) from the drip chamber (59) so as to force exit of said flow of radiopharmaceutical (FR) from the cartridge (1).

## Patentansprüche

1. Kartusche zur Aufnahme eines flüssigen Radiopharmazeutikums und zu dessen Verabreichung durch Infusion an einen Patienten, wobei die Kartusche (1) umfasst: einen Behälter (2) für das Radiopharmazeutikum, der aus Kunststoff hergestellt ist und an seinen beiden longitudinalen Enden jeweils zwei Mündungen (3, 4) aufweist; ein Gehäuse (19) für den Behälter (2), das aus strahlungsabschirmendem Material hergestellt ist; und zwei Stöpsel (5, 6), von denen jeder eine entsprechende Mündung (3, 4) hermetisch verschließt und eine entsprechende Membran (7, 8) aufweist, die von einer Nadel durchstochen werden kann, um es dieser zu ermöglichen, in den Behälter (2) einzudringen, um die Kartusche (1) zu füllen oder zu entleeren; wobei der Behälter (2) außerdem eine Entlüftungsöffnung (15) aufweist, die durch eine hydrophobe Membran (16) verschlossen ist, um während des Befüllens oder Entleerens der Kartusche (1) Luft durchzulassen; wobei das Gehäuse (19) außen an dem Behälter (2) haftet und an seinen beiden longitudinalen Enden jeweils Öffnungen (20, 21) aufweist, die an Positionen ausgebildet sind, die den beiden Mündungen (3, 4) entsprechen, aus denen die Stöpsel (5, 6) zumindest teilweise herausragen.

2. Kartusche nach Anspruch 1, wobei das Entlüftungsöffnung (1) in einer Wand (9a) des Behälters (2) transversal zu einer longitudinalen Achse (2a) des Behälters (2) gemacht ist.

3. Kartusche nach Anspruch 1 oder Anspruch 2, wobei der Behälter (2) wie ein Kolben geformt ist, der einen Boden (9) und einen Hals (10) aufweist, wobei eine erste der Mündungen (3) und die Entlüftungsöffnung (15) in dem Boden (9) und die zweite Mündung (4) mit der Mündung des Halses (10) übereinstimmt.

4. Kartusche nach einem der Ansprüche 1 bis 3, wobei der Behälter (2) in seinem Inneren keinen Stempel oder Schieber aufweist.

5. Abgeschirmter Behälter, der zur Aufnahme einer Kartusche nach einem der Ansprüche 1 bis 4 konfiguriert ist; wobei der abgeschirmte Behälter (22) Folgendes umfasst: einen becherförmigen Stützkörper (23); einen becherförmigen Innenkörper (24), der aus strahlungsabschirmendem Material hergestellt ist, vollständig innen an dem becherförmigen Stützkörper (23) haftet und zur Aufnahme der Kartusche (1) ausgelegt ist, einen Hauptdeckel (25) zum Verschließen des becherförmigen Stützkörpers (23) und einen Innendeckel (26), der aus strahlungsabschirmendem Material besteht und in Bezug auf den Hauptdeckel (25) zum Verschließen des becherförmigen Innenkörpers (26) befestigt ist, wenn der Hauptdeckel (25) den becherförmigen Stützkörper (23) verschließt, wobei der Boden des becherförmigen Innenkörpers (24) und der Innendeckel (26) jeweils erste Öffnungen (33, 34) aufweisen, von denen jede im Gebrauch mit einem jeweiligen der Stöpsel (5, 6) der Kartusche (1) in Eingriff gebracht werden kann, wobei der Boden des becherförmigen Stützkörpers (23) und der Hauptdeckel (25) jeweilige zweite Öffnungen (35, 36) aufweisen, von denen jede im Wesentlichen auf eine jeweilige erste Öffnung (33, 34) ausgerichtet ist, um im Gebrauch den Durchgang einer entsprechenden Nadel zum Einführen derselben in die perforierbare Membran (7, 8) des Stöpsels (5, 6) zu ermöglichen, der mit der ersten Öffnung (33, 34) in Eingriff steht.

6. Abgeschirmter Behälter nach Anspruch 5, mit zwei zusätzlichen Deckeln (37, 38) von denen ein erster (37) an der Außenseite des Bodens des becherförmigen Stützkörpers (23) und der andere an der Außenseite des Hauptdeckels (25) angelenkt ist und die auf ihren eigenen Innenflächen mit entsprechenden Abschnitten (39, 40) aus strahlungsabschirmendem Material versehen sind, von denen jeder den Zweck hat, eine entsprechende der zweiten Öffnungen (35, 36) zu verschließen.

7. Abgeschirmter Behälter nach Anspruch 5 oder Anspruch 6, mit einem ersten Paar von Hebelverschlussmechanismen (45), bei denen die jeweiligen Hebel (45a) an jeweiligen äußeren Oberflächenabschnitten (46) des becherförmigen Stützkörpers (23) angelenkt sind, die in Bezug auf eine longitudinale Achse (22a) des becherförmigen Stützkörpers (23) diametral gegenüberliegen, und bei denen die jeweiligen Haken (45b) an der Außenseite des Hauptdeckels (25) befestigt sind, um letzteren gegen den becherförmigen Stützkörper (23) zu sichern.

8. Abgeschirmter Behälter nach einem der Ansprüche 5 bis 7, wobei die Mündung des becherförmigen Stützkörpers (23) eine erste hermetische Dichtungseinrichtung (29) aufweist, die dazu bestimmt ist, von dem Hauptdeckel (25) gedrückt zu werden, wenn er sich in der Position zum Schließen des becherförmigen Stützkörpers (23) befindet, um eine hermetische Abdichtung zwischen dem Hauptdeckel (25) und dem becherförmigen Stützkörper (23) zu gewährleisten.

9. Abgeschirmter Behälter nach einem der Ansprüche 6 bis 8, wobei jede der zweiten Öffnungen (35, 36) mit entsprechenden hermetischen Dichtungsmitteln (41, 42) versehen ist, die dazu bestimmt sind, von den zusätzlichen Deckeln (37, 38) gedrückt zu werden, wenn sie sich in der Position zum Verschließen der zweiten Öffnungen (35, 36) befinden, um ein hermetisches Verschließen der letzteren zu gewährleisten.

10. Abgeschirmter Behälter nach einem der Ansprüche 6 bis 9, mit einem zweiten Paar von Hebelverschlussmechanismen (47, 48), bei denen die jeweiligen Hebel (47a, 48a) entlang eines weiteren äußeren Oberflächenabschnitts (49) des becherförmigen Stützkörpers (23) ausgerichtet und an diesem angelenkt sind, und den jeweiligen Haken (47b, 48b), von denen jeder an einem jeweiligen zusätzlichen Deckel (37, 38) befestigt ist, um das Schließen der zusätzlichen Deckel (37, 38) zu gewährleisten.

11. Vorrichtung zur Verabreichung einer radiopharmazeutischen Dosis an einen Patienten durch Infusion, umfassend: eine Kartusche nach einem der Ansprüche 1 bis 4, die die radiopharmazeutische Dosis enthält; einen abgeschirmten Behälter (22) nach einem der Ansprüche 5 bis 10, der die Kartusche (1) enthält, eine Stützeinrichtung (51) zum Stützen einer eine Kochsalzlösung enthaltenden Einrichtung (54, 55) und des abgeschirmten Behälters (22); einen ersten Schlauch (62), der mit der eine Kochsalzlösung enthaltenden Einrichtung (55, 54) verbunden und mit einer ersten Nadel (63) versehen ist, die durch die entsprechenden ersten und zweiten Öffnungen (33, 35) des abgeschirmten Behälters (22) in eine erste perforierbare Membran (7) der Kartusche (1) eingeführt wird, um die Kochsalzlösung in die Kartusche (1) zuzuführen; ein zweiter Schlauch (64), der mit einer zweiten Nadel (65) versehen ist, die durch die anderen entsprechenden ersten und zweiten Öffnungen (33, 35) des abgeschirmten Behälters (22) in die andere perforierbare Membran (8) der Kartusche (1) eingeführt wird, um einen Strom von Radiopharmazeutika (FR) abzuziehen; einen Doppeleinlassverbinder (66), der mit dem zweiten Schlauch (64) und mittels eines dritten Schlauchs (61) mit der Kochsalzlösung enthaltenden Einrichtung (54, 55) verbunden ist, um eine Mischung (MX) aus Kochsalzlösung und Radiopharmazeutikum zuzuführen; und eine Tropfkammer (59), die zwischen dem Auslass des Doppeleinlassverbinders (66) und einem venösen Zugang des Patienten angeschlossen ist, um die Mischung (MX) in den Patienten zu infundieren.

12. Vorrichtung nach Anspruch 11, wobei die Kartusche (1) eine Kartusche nach Anspruch 4 ist und die Stützeinrichtung (51) Rückhaltemittel (58) umfasst, um den abgeschirmten Behälter (22) in lösbarer Weise zurückzuhalten, wobei die Kartusche (1) mit dem Hals (10) nach unten gerichtet ist.

13. Vorrichtung nach Anspruch 11 oder Anspruch 12, wobei die Stützeinrichtung (51) die Kochsalzlösung enthaltenden Einrichtung (54, 55) in einer ersten Höhe (H1) von dem Boden und den abschirmten Behälter (22) in einer zweiten Höhe (H2) von dem Boden trägt, wobei die erste Höhe (H1) und die zweite Höhe (H2) größer als die Höhe des venösen Zugangs vom Boden ist und die zweite Höhe (H2) kleiner ist als die erste Höhe (H1), so dass der Fluss des Radiopharmazeutikums (FR) durch das Gewicht der in der Kartusche (1) eintretenden Kochsalzlösung (FS2) aus der Kartusche (1) abfällt.

14. Vorrichtung nach einem der Ansprüche 11 bis 13 umfassend einer Peristaltikpumpe (60), die zwischen dem Auslass der Tropfkammer (59) und dem venösen Zugang angeschlossen ist, um die Mischung (MX) aus der Tropfkammer (59) anzusaugen, um den Austritt des Flusses des Radiopharmazeutikums (FR) aus der Kartusche (1) zu erzwingen.

## Revendications

1. Cartouche pour contenir un produit radiopharmaceutique liquide et pour son administration par perfusion à un patient, la cartouche (1) comprenant : un récipient (2) pour le produit radiopharmaceutique, qui est fait d'une matière plastique et a, à ses deux extrémités longitudinales, deux embouchures (3, 4) respectives ; un boîtier (19) pour le récipient (2), qui est fait d'une matière antirayonnement ; et deux bouchons (5, 6), chacun d'entre eux scellant hermétiquement une embouchure (3, 4) respective et comprenant un diaphragme (7, 8) respectif qui peut être perforé par une aiguille pour permettre à cette dernière de pénétrer dans le récipient (2) afin de remplir ou vider la cartouche (1) ; le récipient (2) ayant par ailleurs un trou de ventilation (15) fermé par une membrane hydrophobe (16) pour le passage d'air durant le remplissage ou le vidage de la cartouche (1) ; le boîtier (19) adhérant extérieurement audit récipient (2) et ayant, à ses deux extrémités longitudinales, des ouvertures (20, 21) respectives faites dans des positions correspondant audites deux embouchures (3, 4), à partir desquelles lesdits bouchons (5, 6) font saillie au moins partiellement.

2. Cartouche selon la revendication 1, dans laquelle ledit trou de ventilation (15) est fait dans une paroi (9a) dudit récipient (2) transversalement par rapport à un axe longitudinal (2a) du récipient (2).

3. Cartouche selon la revendication 1 ou la revendication 2, dans laquelle ledit récipient (2) a la forme d'une fiole qui a un fond (9) et un goulot (10) ; un premier parmi lesdites embouchures (3) et ledit trou de ventilation (15) étant fait dans ledit fond (9) et la seconde embouchure (4) coïncidant avec l'embouchure dudit goulot (10).

4. Cartouche selon l'une quelconque des revendications 1 à 3, dans laquelle ledit récipient (2) est sans aucun piston ni poussoir à l'intérieur de lui.

5. Récipient protégé configuré pour contenir une cartouche selon l'une quelconque des revendications 1 à 4 ; le récipient protégé (22) comprenant : un corps de support en forme de coupe (23) ; un corps intérieur en forme de coupe (24), qui est fait d'une matière antirayonnement, adhère complètement à l'intérieur au corps de support en forme de coupe (23) et est conçu pour abriter la cartouche (1) ; un couvercle principal (25) pour fermer le corps de support en forme de coupe (23), et un couvercle intérieur (26), qui est fait d'une matière antirayonnement et est fixe par rapport au couvercle principal (25) pour fermer le corps intérieur en forme de coupe (26) lorsque le couvercle principal (25) ferme le corps de support en forme de coupe (23) ; le fond dudit corps intérieur en forme de coupe (24) et le couvercle intérieur (26) ayant des premières ouvertures (33, 34) respectives, chacune d'entre elles pouvant être mise en prise, en utilisation, par un respectif parmi lesdits bouchons (5, 6) de la cartouche (1) ; le fond du corps de support en forme de coupe (23) et le couvercle principal (25) ayant des secondes ouvertures (35, 36) respectives, chacune d'entre elles étant sensiblement alignée sur une première ouverture (33, 34) respective pour permettre, en utilisation, le passage d'une aiguille correspondante pour une insertion de celle-ci dans le diaphragme (7, 8) perforable du bouchon (5, 6) qui met en prise la première ouverture (33, 34).

6. Récipient protégé selon la revendication 5, et comprenant deux couvercles auxiliaires (37, 38), qui sont articulés, un premier (37), sur l'extérieur dudit fond du corps de support en forme de coupe (23), et l'autre, sur l'extérieur dudit couvercle principal (25) et sont dotés, sur leurs propres faces intérieures, de parties (39, 40) respectives faites d'une matière antirayonnement, chacune d'entre elles ayant pour but de fermer une respective parmi lesdites secondes ouvertures (35, 36).

7. Récipient protégé selon la revendication 5 ou la revendication 6, et comprenant une première paire de mécanismes de fermeture à leviers (45), qui ont les leviers (45a) respectifs articulés sur des parties de surfaces extérieures (46) respectives dudit corps de support en forme de coupe (23) qui sont diamétralement opposées par rapport à un axe longitudinal (22a) du corps de support en forme de coupe (23) et ont les crochets (45b) respectifs fixés sur l'extérieur dudit couvercle principal (25) pour fixer ce dernier contre le corps de support en forme de coupe (23).

8. Récipient protégé selon l'une quelconque des revendications 5 à 7, dans lequel l'embouchure dudit corps de support en forme de coupe (23) a des premiers moyens de scellement hermétique (29) conçus pour être pressés par ledit couvercle principal (25) lorsqu'il est dans la position pour fermer le corps de support en forme de coupe (23) pour garantir un scellement hermétique entre le couvercle principal (25) et le corps de support en forme de coupe (23).

9. Récipient protégé selon l'une quelconque des revendications 6 à 8, dans lequel chacune desdites secondes ouvertures (35, 36) est dotée de moyens de scellement hermétique (41, 42) respectifs conçus pour être pressés par lesdits couvercles auxiliaires (37, 38) lorsqu'ils sont dans la position pour fermer les secondes ouvertures (35, 36) pour garantir une fermeture hermétique de ces dernières.

10. Récipient protégé selon l'une quelconque des revendications 6 à 9, et comprenant une seconde paire de mécanismes de fermeture à leviers (47, 48), qui ont les leviers (47a, 48a) respectifs alignés le long de, et articulés sur, une partie de surface extérieure (49) supplémentaire du corps de support en forme de coupe (23) et les crochets (47b, 48b) respectifs, chacun d'entre eux étant fixé sur un couvercle auxiliaire (37, 38) respectif pour assurer la fermeture des couvercles auxiliaires (37, 38).

11. Appareil pour l'administration par perfusion d'une dose de produit radiopharmaceutique à un patient, comprenant : une cartouche selon l'une quelconque des revendications 1 à 4 et récipient ladite dose de produit radiopharmaceutique ; un récipient protégé (22) selon l'une quelconque des revendications 5 à 10 et récipient ladite cartouche (1) ; des moyens de support (51) pour supporter des moyens de contenu de solution saline (54, 55) et ledit récipient protégé (22) ; un premier tube (62) raccordé aux moyens de contenu de solution saline (55, 54) et doté d'une première aiguille (63) devant être insérée, à travers les première et seconde ouvertures (33, 35) correspondantes du récipient protégé (22), dans un premier diaphragme (7) perforable de la cartouche (1) pour fournir la solution saline à la cartouche (1) ; un deuxième tube (64) doté d'une seconde aiguille (65) devant être insérée, à travers les autres première et seconde ouvertures (33, 35) correspondantes du récipient protégé (22), dans l'autre diaphragme (8) perforable de la cartouche (1) pour extraire un écoulement de produit radiopharmaceutique (FR) ; un raccord à double entrée (66) raccordé au deuxième tube (64) et, au moyen d'un troisième tube (61), aux moyens de contenu de solution saline (54, 55) pour fournir un mélange (MX) de solution saline et de produit radiopharmaceutique ; et une chambre compte-gouttes (59), raccordée entre l'évacuation du raccord à double entrée (66) et un accès veineux du patient pour la perfusion du mélange (MX) dans le patient.

12. Appareil selon la revendication 11, dans lequel ladite cartouche (1) est une cartouche selon la revendication 4 et lesdits moyens de support (51) comprennent des moyens de retenue (58) pour maintenir, d'une manière libérable, ledit récipient protégé (22), avec ladite cartouche (1) orientée avec ledit goulot (10) faisant face vers le bas.

13. Appareil selon la revendication 11 ou la revendication 12, dans lequel lesdits moyens de support (51) supportent lesdits moyens de contenu de solution saline (54, 55) à une première hauteur (H1) à partir du sol et ledit récipient protégé (22) à une seconde hauteur (H2) à partir du sol, la première hauteur (H1) et la seconde hauteur (H2) étant supérieures à la hauteur à partir du sol dudit accès veineux et la seconde hauteur (H2) étant inférieure à la première hauteur (H1) de sorte que ledit écoulement de produit radiopharmaceutique (FR) tombe de la cartouche (1) en raison du poids de la solution saline (FS2) qui entre dans la cartouche (1).

14. Appareil selon l'une quelconque des revendications 11 à 13 et comprenant une pompe péristaltique (60) raccordée entre l'évacuation de ladite chambre compte-gouttes (59) et ledit accès veineux pour aspirer vers l'intérieur ledit mélange (MX) à partir de la chambre compte-gouttes (59) de manière à forcer la sortie dudit écoulement de produit radiopharmaceutique (FR) à partir de la cartouche (1).
